# EUROPEAN PATENT SPECIFICATION

(11) **EP 1 397 125 B1**
(45) Date of publication and mention of the grant of the patent: **30.03.2005**
(21) Application number: 02741904.3
(22) Date of filing: 07.06.2002
(51) Int. Cl.: A61K 31/09, A61K 9/20

(54) **COMPRESSIBLE GUAIFENESIN COMPOSITIONS AND METHOD FOR PRODUCING THEM**
VERPRESSBARE GUAIFENESIN ZUSAMMENSETZUNGEN UND DEREN HERSTELLUNGSVERFAHREN
COMPOSITIONS DE GUAIFENESINE COMPRESSIBLES,et son PROCEDE de fabrication

(30) Priority: 12.06.2001 US 879320
(43) Date of publication of application: 17.03.2004
(73) Proprietor: Rhodia, Inc., Cranbury, New Jersey 08512 (US)
(72) Inventor: UPADHYAY, Ajay, Hasmukhlal, Sayreville, NJ 08872 (US)
(74) Representative: Kyle, Diana
(86) International application number: PCT/US2002/018094
(87) International publication number: WO 2002/100389

(56) References cited:
- WO-A-01/82895
- WO-A-84/04674

## Description

### Field of the Invention

This invention relates to compressible guaifenesin compositions, that is, guaifenesin compositions capable of being compressed to form guaifenesin dosage forms.

### Background of the Invention

Generally there are four methods in use in the United States for manufacture of tablets, namely direct compression, dry powder blend, precompressed dry powder blend and wet granulation, as explained in US Patent 4,439,453. The direct compression method is generally preferred.

In the direct compression method, all the required tableting ingredients are incorporated into a free flowing composition. The composition requires no pre-processing or blending with additional aids in order to be tableted. Rather, the free flowing granulation supplied to the tablet manufacturer can be charged directly to a tableting press. For example, the analgesic aspirin is generally tableted using a direct compression method, since crystalline aspirin is soft and exhibits good plasticity/elasticity when compacted into tablets.

However, guaifenesin itself is generally considered to be non-compressible and not readily amendable to production of directly compressible granulations thereof. Compressible guaifenesin compositions comprising particles of guaifenesin and a binder have been developed and are commercially available. However, known compressible guaifenesin compositions have proven to be highly sensitive to the processing conditions when used to make compressed dosage forms. Such compositions can be used only within a relatively narrowly defined critical range of compression forces to make compressed dosage forms of acceptable quality. Dosage forms made from such compositions tend to exhibit unacceptably high friability and unacceptably low hardness if the compression forces lower than the critical range are used and tend to exhibit "capping", that is, cracking and separation of a part of the dosage form from the body of the dosage form, if compression forces higher than the critical range are used. Known compressible guaifenesin compositions also tend to exhibit inadequate flow properties, that is, such compositions tend to flow relatively slowly or only with the aid of agitation through mechanical vibration, which slows the production of compressed dosage forms from such compositions.

What is needed in the art is a guaifenesin composition that exhibits robustness and flexibility with regard to processing conditions and that can be processed under relatively low compressive forces to produce compressed dosage forms that exhibit low friability, high hardness and that are resistant to capping under relatively high compressive forces. Compressible guaifenesin compositions that exhibit improved flow properties and allow more rapid processing of the composition, such as, for example, more rapid flow of the composition allows more rapid transfer of the composition into compression machinery for making compressed dosage forms, are also highly desirable.

### Summary of the Invention

In a first aspect, the present invention is directed to a guaifenesin composition, comprising guaifenesin and a binder and being in the form of particles, wherein by sieve analysis, based on the total weight of the composition, less than about 30 percent by weight of the particles exhibit a particle size of greater than about 425 micrometers and greater than about 80 percent by weight of the particles exhibit a particle size of greater than about 45 micrometers.

The guaifenesin composition of the present invention provides improved robustness and flexibility with regard to the processing conditions used to produce compressed dosage forms from such compositions, can be processed under relatively low compressive forces to produce compressed dosage forms that exhibit low friability and high hardness and are resistant to capping under relatively high compressive forces. The guaifenesin composition of the present invention also provides improved flow properties and allows more rapid processing of the composition, that is, more rapid transfer of the composition into compression machinery.

In a second aspect, the present invention is directed to a method for making a guaifenesin composition, comprising:
mixing a mixture comprising guaifenesin, a binder and water to form agglomerates;
drying the agglomerates to form dried particles;
classifying the dried particles into first particles having particle sizes less than or equal to a selected classification limit and second particles having particle sizes greater than the classification limit;
milling the second dried particles to reduce their sizes to less than the classification limit; and
combining the milled second particles with the first particles.

In another aspect, the present invention is directed to a method for making a guaifenesin dosage form, comprising compressing a guaifenesin composition, said composition comprising guaifenesin and a binder and being in the form of particles, wherein by sieve analysis, based on the total weight of the composition, less than about 30 percent by weight of the particles exhibit a particle size of greater than about 425 micrometers and greater than about 80 percent by weight of the particles exhibit a particle size of greater than about 45 µm.

### Detailed Description of Invention and Preferred Embodiments

In a preferred embodiment, less than about 25 percent by weight (wt%) of the particles exhibit a particle size of greater than about 425 µm.

In a preferred embodiment, greater than about 85 wt% of the particles exhibit a particle size of greater than about 45 µm.

In a preferred embodiment, from 10 to 60 percent by weight of the particles exhibit a particle size of from greater than 45 µm to less than 150 µm. In a more highly preferred embodiment, from 15 to 55 wt%, even more preferably from 17 to 55 wt%, of the particles exhibit a particle size of from greater than 45 µm to less than 150 µm.

In a preferred embodiment, the guaifenesin composition comprises guaifenesin, a binder, and a solubilizer. Optionally, a disintegrant may be substituted for all or part of the solubilizer.

In a more highly preferred embodiment, the guaifenesin composition is a directly compressible "drum-to-hopper" guaifenesin composition that is capable of being directly compressed into a compressed dosage form, such as, for example, a tablet or a caplet, without addition of other components. In a highly preferred embodiment, the directly compressible drum-to-hopper guaifenesin composition comprises guaifenesin, a binder, a solubilizer, a glidant and a lubricant. Optionally, a disintegrant may be substituted for all or part of the solubilizer. In a more highly preferred embodiment, the directly compressible drum-to-hopper compressible guaifenesin composition comprises, based on the total weight of dry ingredients:
from 85 to 97.5 wt%, more preferably from 90 to 97 wt% and even more preferably from 92 to 96.5 wt%, guaifenesin,
from 1.0 to 7 wt%, more preferably from 1.5 to 5 wt% and even more preferably from 2 to 4 wt%, of the binder,
from 0.2 to 4 wt%, more preferably from 0.4 to 3 wt% and even more preferably from 0.5 to 2 wt%, of the solubilizer and/or disintegrant,
from 0.1 to 2 wt%, more preferably from 0.2 to 1.8 wt% and even more preferably from 0.25 to 1.5 wt%, of the glidant, and
from 0.1 to 2 wt%, more preferably from 0.2 to 1.8 wt% and even more preferably from 0.25 to 1.5 wt%, of the lubricant.

The guaifenesin component of the composition of the present invention may be any particulate pharmaceutically acceptable guaifenesin. In a preferred embodiment, the guaifenesin is particulate USP grade guaifenesin. Preferably, by sieve analysis, based on the total weight of the particles, greater than about 10 wt% of the particles exhibit a particle size of greater than 75 µm and greater than about 55 wt% of the particles exhibit a particle size of greater than 45 µm.

Any pharmaceutically acceptable compound capable of rendering the guaifenesin particles compactable is suitable as the binder compound of the composition of the present invention. Suitable binder compounds are known in the art and include, for example, polyvinylpyrrolidones, hydroxyalkyl cellulose derivatives such as, hyrdroxypropyl methylcellulose, hydroxypropyl cellulose and hydroxyethyl cellulose as well as mixtures thereof. In a preferred embodiment, the binder compound comprises a polyvinylpyrrolidone. Suitable polyvinylpyrrolidones are, for example, those having a weight average molecular weight (M_{w}) of from 25,000 to 1,500,000. In a preferred embodiment, the polyvinylpyrrolidone has a weight average molecular weight of from 40,000 to 60,000.

Any pharmaceutically acceptable solubilizer known in the art is suitable as the solubilizer component of the composition of the present invention. In a preferred embodiment, the solubilizer comprises a pharmaceutically acceptable polysaccharide. Suitable polysaccharide solubilizers include, for example, maltodextrin, sorbitol, lactose. In a highly preferred embodiment, the polysaccharide solubilizer comprises a maltodextrin, more preferably, a maltodextrin having a dextrose equivalent value of from 4 to 20.

Any pharmaceutically acceptable compound that is substantially insoluble in water but capable of swelling in water in order to accelerate the disintegration and dissolution in an aqueous medium of compressed dosage forms formed from the composition of the present invention is suitable as the disintegrant of the composition of the present invention. Suitable disintegrants are known in the art and include, for example, sodium carboxylmethyl starch, also known as sodium starch glycolate, microcrystalline cellulose, soy protein, alginic acid, crosslinked polyvinylpyrrolidone, also known as crosslinked povidone and crosslinked sodium carboxymethylcellulose, also known as croscarmellose sodium, as well as mixtures thereof. Preferably, the disintegrant comprises croscarmellose sodium.

The glidant component of the composition of the present invention serves as a densifier, flow aid and moisture scavenger. Pharmaceutically acceptable glidants that are suitable for use in the composition of the present invention, such as, for example, silica, talc, tricalcium phosphate, are known in the art. In a preferred embodiment, a silica is used as the glidant. Suitable silicas include precipitated silicas and fumed silicas.

Any pharmaceutically acceptable lubricant is suitable as the lubricant of the composition of the present invention. Suitable lubricants are known in the art and include, for example, stearic acid or mixtures of fatty acids, hydrogenated vegetable oils, triglycerides of fatty acids, metal stearates, such as for example, zinc stearate and magnesium stearate, or metal salts of fatty acid mixtures, sodium lauryl sulfate, polyethylene glycol and talc, as well as mixtures thereof. Preferably, the lubricant comprises stearic acid.

In a preferred embodiment, a high shear mixer equipped with a high shear impeller and a chopper is used to provide high shear mixing. Vertical high shear mixers and horizontal high shear mixers are each suitable as the high shear mixer. A vertical high shear mixer is preferred, more preferably, a vertical high shear mixer with a top driven or bottom driven impeller and one or more choppers having from 2 to 12 blades per chopper. Guaifenesin exhibits a relatively low melting point, that is, a melting point of from 78°C to 82°C. Mixing in a vertical high shear mixer presents less risk of overheating and degradation of the guaifenesin composition during mixing than does mixing in a horizontal high shear mixer.

In a preferred embodiment, the guaifenesin is charged to a mixer and mixed under conditions effective to distribute particles of the guaifenesin powder within the mixer. In a preferred embodiment, the guaifenesin is mixed at high impeller and chopper speeds, such as, for example, in large scale manufacture of about 400 kg of granulated product, an impeller speed of greater than 50 revolutions per minute (rpm) and a chopper speed of greater than 800 rpm, for from 1 to 10 minutes.

In a preferred embodiment, once the guaifenesin particles are distributed in the mixer, the binder, a solubilizer or disintegrant and water are then charged to the mixer. Preferably, the amount of water used ranges from 2 to 20 more preferably from 5 to 15 parts by weight (pbw) water per 100 pbw of the combined amount of guaifenesin, binder and solubilizer. In a preferred embodiment, the binder, solubilizer and water are combined prior to addition to the mixer. The guaifenesin, binder, solubilizer and water are then mixed under conditions effective to distribute the binder, solubilizer and water in the guaifenesin powder and form a wet mass. Disintegrant, if used, may be added, in dry form, in the mixing step, in a subsequent blending step or in both the mixing step and a subsequent blending step. If a disintegrant is to be added to the composition during the mixing step, then dry disintegrant should be added to the guaifenesin powder prior to the addition of the water and binder, or alternatively, charged to the mixer after addition of the water and binder. In a preferred embodiment, the mixing is continued for a time period effective to produce agglomerates of guaifenesin powder, binder, solubilizer and/or disintegrant and water. In a preferred embodiment, the mixing is continued until a sample of the agglomerates can be compressed by hand to form a cohesive mass. Suitable mixing conditions are high shear conditions, such as, for example, mixing under high impeller and chopper speeds. For example, in large scale manufacture of about 400 kilograms (kg) of granulated product, an impeller speed of greater than 50 rpm and a chopper speed of greater than 800 rpm, of from 1 to 30 minutes, more preferably from 5 to 25 minutes.

In a preferred embodiment, the wet mass of agglomerates produced in the above described mixing step is then wet milled under conditions effective to reduce the size of the agglomerates. In a highly preferred embodiment, the mixture is wet milled in a conical mill using a round or square impeller at a moderate to high impeller speed, such as, for example from 250 rpm to 1500 rpm. In one embodiment, the mixture is wet milled without using a screen. More preferably, the mixture is wet milled at moderate to high impeller speed using a screen having round or square openings of greater than about 9 square millimeters (mm²) in size. In a preferred embodiment, the wet mixture is transferred from the high shear mixer to the conical mill by vacuum.

In a preferred embodiment, the wet milled mixture is dried at low temperature, preferably at a temperature of less than 65°C. The relatively low drying temperature avoids overheating the guaifenesin component of the guaifenesin composition and avoids undesirably rapid drying and hardening of the surfaces of the particles, which would slow transfer of moisture from the interior of the particle and lead to inadequate drying. In a highly preferred embodiment, the wet milled mixture is dried at a temperature from 20°C to 65°C, more preferably from 20°C to 40°C. The drying may be accomplished using equipment known in the art. Such as, for example, a fluidized bed or an oven equipped with drying trays. In a preferred embodiment, the drying step of the process of the present invention is conducted in a fluidized bed.

In a highly preferred embodiment, the wet milled mixture is dried in a fluidized bed at low temperature, preferably at a temperature of less than 65°C, with a relatively high flow rate of fluidizing gas, for example, in large scale manufacture of about 400 kg of granulated product, a gas flow rate of from 36,8-142 m³ (1300 to 5000 cubic feet) per minute (cfm), more preferably from 36,8 to 127 m³ (1300 to 4500 cfm). The gas flow rate may be increased as the particles dry. In a preferred embodiment, air is used as the fluidizing gas.

In a preferred embodiment, the wet milled mixture is dried to a moisture content, based on the total weight of dried mixture, of less than about 1.5 wt% water, more preferably less than about 1.2 wt%, even more preferably less than about 1.0 wt% water, and still more preferably less than 0.85 wt% water, based on the total weight of dried composition.

In a preferred embodiment, the dried mixture is classified into first particles having a particle size of less or equal to a selected classification limit and second particles having a particle size greater than the classification limit. The second particles are dry milled, for example in a conical mill, to reduce their particle size to less than or equal to the classification limit and the dry milled particles are then blended with the first particles.

In a preferred embodiment, the second particles are dry milled in a conical mill using a square or round impeller at a low to moderate speed, such as for example, from 500 to 1000 rpm, and using a screen having openings of from 1.0 mm to 2.0 mm.

For a particular guaifenesin composition, the classification limit is selected by an iterative process in order to obtain a desired particle size distribution in the finished product. In a preferred embodiment, the classification limit is selected to be a value of greater than or equal to 850 µm, such as, for example, 850 µm (corresponding to a US 20 mesh screen), 1.0 mm (corresponding to a US 18 mesh screen), 1.18 mm (corresponding to a US 16 mesh screen) or 1.40 mm (corresponding to a US 14 mesh screen). In a highly preferred embodiment, the classification limit is 1.18 mm. In general, compositions in which a relatively high amount of binder is used tend to include a higher amount of relatively large particles and require setting a relatively low classification limit in order to bring the particle size distribution into the desired range, and compositions in which a relatively low amount of binder is used tend to include a higher amount of relatively small particles and require setting a relatively high classification limit in order to bring the particle size distribution into the desired range.

In the above described directly compressible drum-to-hopper embodiment of the guaifenesin composition of the present invention, a glidant and a lubricant, and, optionally, a disintegrant, are added to particles of the dried mixture and mixed, for example, using a V-blender, to make a directly compressible, drum-to-hopper guaifenesin composition.

In a preferred embodiment, the composition of the present invention comprises a moisture content, as measured by Karl Fischer titration, of up to about 1.5 wt%, more preferably up to about 1.2 wt% and even more preferably from 0.3 to 1.0 wt%.

In a preferred embodiment, the guaifenesin composition of the present invention exhibits a density, as measured using a VanKel cylinder, of from 0.40 to 0.70 grams per cubic centimeter (g/cc), more preferably from 0.50 to 0.60 g/cc.

In a preferred embodiment, the guaifenesin composition of the present invention exhibits a flow rate of greater than or equal to 6.5 grams per second (g/sec), more preferably greater than or equal to 7.0 g/sec, and even more preferably greater than or equal to 7.5 g/sec as measured using a VanKel flowmeter, preferably without requiring vibration to induce flow.

The guaifenesin composition may, optionally, further comprise minor amounts of other conventional pharmaceutically acceptable excipients and additives known in the art, such as, for example, surfactants, such as, for example, sodium lauryl sulfate, or suitable amounts of sustained release additive, such as for example, hydroxypropyl methylcellulose, ethylcellulose, hydroxypropyl cellulose, xanthan gum, sodium alginate.

The guaifenesin composition may, optionally, be blended with one or more co-active components, such as for example, analgesics, decongestants, antitussives, antihistamines, cough suppressants and/or other expectorants. Suitable co-active components include, for example, one or more of pseudoephedrine hydrochloride, dextromethorphan hydrobromide, chlorpheniramine maleate and acetaminophen. In preferred embodiment, the present invention is directed to a compressible composition comprising from 1 wt% to 99 wt%, more preferably from 25 wt% to 99 wt%, of the above described guaifenesin composition of the composition and from 1 wt% to 99 wt%, more preferably from 1 wt% to 75 wt%. of a co-active ingredient.

The fully formulated, i.e., drum -to-hopper, directly compressible embodiment of the composition of the present invention is useful for making finished oral dosage forms, such as, for example, tablets and caplets, by conventional methods. Such tablets contain all the ingredients required to pass the applicable US Pharmacopeia requirements, such as, for example, USP dissolution, so that they are be suitable for sale in the consumer marketplace.

In a preferred embodiment, a compressed guaifenesin dosage form made by compressing the compressible guaifenesin composition of the present invention at a compressive force of less than or equal to 1.5 tons exhibit a hardness of greater than 15.0 kiloponds (kp), more preferably greater than or equal to 15.5 kp, even more preferably greater than 16.0 kp, as measured using a Schleuniger hardness tester.

In a preferred embodiment, compressed guaifenesin dosage forms made by compressing the compressible guaifenesin composition of the present invention at a compressive force of less than or equal to 2.5 tons exhibit substantially no capping.

In a preferred embodiment, compressed guaifenesin dosage forms made by compressing the compressible guaifenesin composition of the present invention at a compressive force of from 0.5 ton to 2.5 tons exhibit less than 1.0%, more preferably less than 0.8%, friability.

The invention is illustrated by the following examples. Unless specifically noted otherwise, all percent by weight (wt%) values in all examples are based on total weight of dry ingredients.

USP grade guaifenesin powders GP-A, GP-B, GP-C and GP-D having the respective particle size distributions set forth below, were used in the examples. Guaifenesin powder GP-A was made by Rhodia Inc., guaifenesin powders GP-B, GP-C and GP-D were obtained from other commercial sources. In the tables below, the result "%R" for a sample means the amount of sample retained on the corresponding screen size set forth in the table, expressed as a percent by weight of the total sample amount, and the result "%CR" for a sample means the cumulative amount of the sample retained on all screen sizes larger than the corresponding screen size, expressed as a percent by weight of the total sample amount.

| Guaifenesin Powder Particle Size Distributions | | | | | | | | |
|---|---|---|---|---|---|---|---|---|
| | GP-A | | GP-B | | GP-C | | GP-D | |
| US Mesh | % R | % CR | % R | % CR | % R | % CR | % R | % CR |
| 20 (850um) | 0 | 0 | 0 | 0 | 0 | 0 | 0 | 0 |
| 40 (425um) | 0 | 0 | 0.4 | 0.4 | 2.0 | 2.0 | 0 | 0 |
| 60 (250um) | 0.8 | 0.8 | 0 | 0.4 | 1.2 | 3.2 | 0 | 0 |
| 80 (180um) | 2.4 | 3.2 | 2.0 | 2.4 | 0 | 3.2 | 3.2 | 3.2 |
| 100 (150um) | 4.4 | 7.6 | 0.8 | 3.2 | 0.8 | 4.0 | 6.4 | 9.6 |
| 200 (75um) | 30.8 | 38.4 | 8.0 | 11.2 | 7.2 | 11.2 | 50.4 | 60.0 |
| 325 (45um) | 38.4 | 76.8 | 46.8 | 58.0 | 66.4 | 77.6 | 29.6 | 89.6 |
| pan (<45um) | 23.2 | 100 | 42.0 | 100 | 22.4 | 100 | 10.4 | 100 |
| Bulk density. (g/cc) | 0.45 | | 0.41 | | 0.35 | | 0.40 | |

### Examples 1 and 2

A 6 kg batch of the guaifenesin composition of Example 1 was made as follows. Guaifenesin (5.700 kg (95 wt%) of guaifenesin powder GP-A) was mixed in a top driven vertical high shear mixer (12.5 liter working capacity, equipped with impeller and a 2 blade chopper (GRAL-25, Colette Machines, GEA Group)) for 1 minute using a main blade speed of 295 rpm at 0.5 kW and a chopper speed of 1460 rpm at 0.5 kW. A binder mixture (0.180 kg (3 wt%) Povidone K-30, 0.060 kg (1 wt%) Maltodextrin MD040 and 0.720 kg water) was added to the guaifenesin at the rate of 128 g/min over about 7 minutes and 30 seconds, with continued mixing at a main blade speed of 295 rpm at 0.8 kW and a chopper speed of 1460 rpm at 0.8 kW. After the addition of binder mixture was completed, mixing was continued for one minute at a main blade speed of 295 rpm at 0.8 kW and a chopper speed of 1460 rpm at 0.8 kW and then for an additional two minutes at a main blade speed of 295 rpm at 0.8 kW and a chopper speed of 2870 rpm at 0.8 kW.

The mixture was manually transferred from the vertical mixer to a conical mill (Quadrol Comil 197) and wet milled using a round impeller rotating at 690 rpm and a screen with 9.5 mm square openings. The wet-milled mixture was dried in a fluid bed drier (Glatt WSG-5, Glatt GmbH) at an inlet temperature of 25-48°C, a product temperature of 23-25°C and an air volume of 30% to a moisture content of 0.68 wt%, based on the total weight of dried mixture.

The dried mixture was then dry milled in a mill (GLT001 Oscillator, Frewitt) using a screen opening size of 850 µm and an oscillating rotor at 200-300 cycles per minute.

A glidant (0.030 kg (0.5 wt%) of Syloid 244 FP) and a lubricant (0.030 kg (0.5 wt%) of stearic acid) were added to the milled mixture and mixed in a 16 quart V-blender for 15 minutes.

The composition of Example 2 was made substantially according to the process used in Example 1, except that:
2(i) the binder mixture contained 0.120 kg (2 wt%) Povidone K-30, 0.060 kg (1 wt%) Povidone K-90, 0.060 kg (1 wt%) Maltodextrin MD040 and 0.8750 kg water,
2(ii) the binder mixture was added at a rate of 135 g/min,
2(iii) after addition of the binder mixture, mixing was continued for one minute at a main blade speed of 295 rpm at 1.0 kW and a chopper speed of 1460 rpm at 1.0 kW and then for an additional two minutes at a main blade speed of 295 rpm at 1.1 kW and a chopper speed of 2870 rpm at 1.1 kW, and
2 (iv) the wet milled mixture was dried to a moisture content of 0.75%.

### Examples 3-7

A 430 kg batch of the composition of Example 3 was made as follows. Guaifenesin (408.50 kg (95 wt%) of Guaifenesin powder GP-A) was mixed in a bottom driven vertical high shear mixer (having a working capacity of from 400 to 1000 liters and equipped with an impeller and two 12 blade choppers, (Powerex VG-1500, Glatt GmbH)) for 1 minute using a main blade speed of about 70 rpm at 4.4 kW and a chopper speed of about 1100-1125 rpm at 1.4 kW. A binder mixture (12.9 kg (3 wt%) Povidone K-30, 4.3 kg (1 wt%) Maltodextrin MD040 and 51.6 kg water) was added to the guaifenesin at the rate of 3.4 - 4 kg/min over about 20 minutes, with continued mixing at a main blade speed of 70 rpm at 14.3 kW and a chopper speed of about 1100 -1125 rpm at 1.7 kW. The temperature of the mixture did not exceed about 38°C.

The mixture was manually transferred from the vertical mixer to a conical mill (Glatt Sieve 300, Glatt GmbH) and then wet milled using a round impeller at 350 rpm, with no screen. The wet-milled mixture was dried in a fluid bed drier (Glatt GPCG300, Glatt GmbH) at an inlet temperature of 38-60°C, a product temperature of 27-33°C and an air volume of about 2900-3900 cfm to a moisture content of 0.66%, based on the total weight of dried mixture.

The dried mixture was dry milled in a conical mill (Glatt Sieve 180, Glatt GmbH) using a screen opening size of 1.0 mm and an oscillating rotor at 700 cycles per minute.

A glidant (2.13 kg (0.5 wt% based on total dry ingredients) of Syloid 244 FP) and a lubricant (2.13 kg (0.5 wt% based on total dry ingredients) of stearic acid) were added to the milled mixture and mixed in a 40 ft³ V-blender for 15 minutes.

The composition of example 4 was made substantially according to the process used in Example 3, except that:
4(i) four batches of 425.7 kg each were made separately and then combined as noted in 4(v) below to form the 1720 kg batch of Example 4,
4(ii) for each of the four batches, the mixture of guaifenesin, binder, solubilizer and water was wet milled using a conical mill (Quadro Comil 196S) at 900 rpm using a square impeller and a screen with an opening size of 1 cm,
4(iii) for each of the four batches, the wet milled mixture was dried to a moisture content of 0.6-1.0%,
4(iv) for each of the four batches, the dried mixture was classified using a vibrator (Sweco) equipped with either a US 16 mesh screen or a US 18 mesh screen into first particles having particle sizes less than or equal to the respective US 16 mesh or US 18 mesh classification limit and second particles having particle sizes of greater than the respective classification limit, only those the particles of each batch that were larger than the classification limit for the batch were dry milled in a conical mill (Glatt Sieve (180)) using a round impeller rotating at 700 rpm and a screen having 1.0 mm openings and the dry milled particles of each batch were then combined with the particles of the batch having a particle size of less than the classification limit for the batch, and
4(v) the four batches were combined, 8.52 kg (0.5 wt%) glidant and 8.52 kg (0.5 wt%) lubricant were added and the combined batches, glidant and lubricant were mixed in a 100 ft³ V blender for 10 minutes.

The composition of example 5 was made substantially according to the process used in Example 3, except that:
5(i) four batches of 425.7 kg kilograms each were made separately and then combined as noted in 5(v) below to form the 1720 kg batch of Example 5,
5(ii) for each of the four batches, the guaifenesin was mixed in the vertical mixer for 10 minutes prior to addition of the binder mixture,
5(ii) for each of the four batches, the mixture of guaifenesin, binder, solubilizer and water was vacuum transferred from the vertical high shear mixer to a conical mill (Glatt Sieve 300) and wet milled at 1250 rpm using a round impeller and a screen with 1.0 cm square openings,
5(iii) for each of the four batches, the wet milled mixture was dried to a moisture content of 0.4-0.9%,
5(iv) for each of the four batches, the dried mixture was classified using a vibrator (Sweco) equipped with a US 16 mesh screen into first particles having particle sizes less than or equal to the US 16 mesh classification limit and second particles having particle sizes of greater than the US 16 mesh classification limit, the particles of each batch that were larger than the classification limit were dry milled in a conical mill (Quadro Comil 196S) using a square impeller rotating at 900 rpm and a screen having 1.0 mm openings and the dry milled particles were then combined with the particles of the batch having a particle size of less than the classification limit, and
5(v) the four batches were combined, 8.52 kg (0.5 wt%) glidant and 8.52 kg (0.5 wt%) lubricant were added and the combined batches, glidant and lubricant were mixed in a 100 ft³ V blender for 10 minutes.

The composition of example 6 was made substantially according to the process used in Example 3, except that:
6(i) four batches of 425.7 kg each were made separately and then combined as noted in 6(vi) below to form the 1720 kg batch of Example 6,
6(ii) for each of the four batches, the binder mixture contained 38.40 kg water and the guaifenesin powder was mixed in the vertical high shear mixer for 10 minutes prior to addition of the binder mixture,
6(iii) for each of the four batches, the mixture of guaifenesin, binder, solubilizer and water was vacuum transferred from the vertical high shear mixer to a conical mill (Glatt Sieve 300) and then wet milled at 1250 rpm using a round impeller and a screen with 1.0 cm square openings,
6(iv) for each of the four batches, the wet milled mixture was dried to a moisture content of 0.6 -0.9%,
6(v) for each of the four batches, the dried mixture was classified using a vibrator (Sweco) equipped a US 16 mesh screen into first particles having particle sizes less than or equal to the US 16 mesh classification limit and second particles having particle sizes of greater than the US 16 mesh classification limit, the particles of each batch that were larger than the classification limit were dry milled in a conical mill (Quadro Comil 196S) using a square impeller rotating at 900 rpm and a screen having 1.7 mm openings and the dry milled particles were then combined with the particles of the batch having a particle size of less than the classification limit, and
6(vi) the four batches were combined, 8.52 kg (0.5 wt%) glidant and 8.52 kg (0.5 wt%) lubricant were added and the combined batches, glidant and lubricant were mixed in a 100 ft³ V blender for 10 minutes.

The composition of example 7 was made substantially according to the process used in Example 3, except that:
7(i) the binder mixture contained 30.0 kg water,
7(ii) the binder was added to the mixture at a rate of about 13.2 kg/min over a time period of about 4 minutes,
7(iii) the mixing was continued for 30 seconds after the addition of the binder mixture was completed,
7(iv) cooling water was circulated in the cooling jacket of the vertical mixer to control the temperature of the mixture (the temperature of the mixture did not exceed 12°C),
7(v) the wet milled mixture was dried to a moisture content of 0.6%, and
7(vi) the dried mixture was classified using a vibrator (Sweco) equipped a US 16 mesh screen into first particles having particle sizes less than or equal to the US 16 mesh classification limit and second particles having particle sizes of greater than the US 16 mesh classification limit, the particles that were larger than the classification limit were dry milled in a conical mill (Quadro Comil 196S) using a square impeller rotating at 900 rpm and a screen having 1.7 mm openings and the dry milled particles were then combined with the particles of the batch having a particle size of less than the classification limit.

### Examples 8-10

A 6 kg batch of the composition of Example 8 was made as follows. Guaifenesin (5.700 kg (95 wt%) of a mixture of guaifenesin powder GP-A and GP-C) was mixed in a bottom driven vertical high shear mixer (Sejong SM-15) for 9 minutes using a main blade speed of 600 rpm at less than 2 amps and a chopper speed of about 1750 rpm at less than 2 amps. A binder mixture (0.180 kg (3 wt%) Povidone K-30, 0.60 kg (1 wt%) Maltodextrin MD040 and 0.72 kg water) was added to the guaifenesin at the rate of about 180 g/min over about 6 minutes, with continued mixing.

The mixture was manually transferred from the vertical mixer to a conical mill (Quadro Comil 197) and wet milled using a square screen opening size of 9.5 mm and a round impeller at 40% of maximum speed. The wet-milled mixture was dried in a fluid bed drier (Glatt WSG-5) at an inlet temperature of 30-40°C, a product temperature of 20-25°C and an air volume of about 40% of maximum to a moisture content of 0.75%, based on the total weight of dried mixture.

The dried mixture was classified using a vibrator (Sweco) equipped with a US 16 mesh screen into first particles (particle sizes less than or equal to the US 16 mesh classification limit) and second particles (particle sizes greater than the US 16 mesh classification limit). Particles having a particle size greater than the classification limit were milled in a dry mill (Quadro Comil 197) using a round impeller at 40% of maximum speed and a screen opening size of 1.7 mm and then blended with the particles having a particle size of less than or equal to the classification limit.

A glidant (0.030 kg (0.5 wt%) of Syloid 244 FP) and a lubricant (0.030 kg (0.5 wt%) of stearic acid) were added to the milled mixture and mixed in a 16 quart V-blender for 15 minutes.

The composition of example 9 was made substantially according to the process used in Example 8, except that:
9(i) guaifenesin Powder GP-B was used,
9(ii) the guaifenesin powder was mixed in the vertical high shear mixer for 2 minutes prior to adding the binder mixture, and
9(iii) the wet milled mixture was dried to a moisture content of 0.5%.

The composition of example 10 was made substantially according to the process used in Example 8, except that:
10(i) guaifenesin Powder GP-D was used,
10(ii) the guaifenesin powder was mixed in the vertical high shear mixer for 2 minutes prior to adding the binder mixture,
10(iii) the binder mixture included 0.78 kg water and was fed into the vertical mixer at a rate of about 115 g/min, and
10(iv) the wet milled mixture was dried to a moisture content of 0.78%.

The particle size distribution, bulk density, moisture content and flow properties were measured for each of the compositions of Examples 1-10 and for Comparison Examples C1-C4. Comparison Examples C1-C4 were commercially available directly compressible guaifenesin compositions. Particle size distribution was measured by sieve analysis using screens of the indicated mesh sizes. Bulk density was measured using a VanKel cylinder. Moisture content was measured by Karl Fischer titration. Flow properties were measured using a VanKel flowmeter with a strip chart recorder. The composition of Comparative Example C3 required vibration to induce flow. Results are given below in TABLES 1A, 1B and 1C

**TABLE 1 A**

| | EX 1 | | Ex 2 | | Ex 3 | | Ex 4 | | Ex 5 | |
|---|---|---|---|---|---|---|---|---|---|---|
| US Mesh | % R | % CR | % R | % CR | % R | % CR | % R | % CR | % R | %CR |
| 20 (850um) | 0 | 0 | 0 | 0 | 0.8 | 0.8 | 1.6 | 1.6 | 3.2 | 3.2 |
| 40 (425um) | 19.6 | 19.6 | 24.0 | 24.0 | 16.4 | 17.2 | 14.8 | 16.4 | 16.0 | 19.2 |
| 60 (250um) | 26.0 | 45.6 | 19.6 | 43.6 | 13.2 | 30.4 | 10.8 | 27.2 | 15.2 | 34.4 |
| 80 (180um) | 19.2 | 64.8 | 14.8 | 58.4 | 12.0 | 42.4 | 12.0 | 39.2 | 13.6 | 48.0 |
| 100 (150um) | 9.2 | 74.0 | 8.0 | 66.4 | 7.6 | 50.0 | 9.6 | 48.8 | 8.4 | 56.4 |
| 200 (75um) | 20.4 | 94.4 | 19.6 | 86.0 | 31.6 | 81.6 | 40.0 | 88.0 | 28.0 | 84.4 |
| 325 (45um) | 0 | 94.4 | 0 | 86.0 | 13.2 | 94.8 | 8.0 | 96.0 | 11.2 | 95.6 |
| pan (<45um) | 5.6 | 100 | 14.0 | 100 | 5.2 | 100 | 3.6 | 99.6 | 4.4 | 100 |
| Bulk Density g/cc | 0.56 | | 0.59 | | 0.61 | | 0.64 | | 0.64 | |
| Moisture % | 0.80 | | 0.78 | | 0.7 | | 0.64 | | 0.43 | |
| Flow g/sec | 9.0 | | 7.1 | | 8.5 | | 9.9 | | 8.3 | |

**TABLE 1B**

| | Ex 6 | | Ex 7 | | Ex 8 | | Ex 9 | | Ex 10 | |
|---|---|---|---|---|---|---|---|---|---|---|
| US Mesh | % R | % CR | % R | %CR | % R | % CR | % R | % CR | % R | % CR |
| 20 (850um) | 2.0 | 2.0 | 1.6 | 1.6 | 1.6 | 1.6 | 4.0 | 4.0 | 1.2 | 1.2 |
| 40 (425um) | 10.8 | 12.8 | 6.8 | 8.4 | 8.0 | 9.6 | 11.6 | 15.6 | 4.8 | 6.0 |
| 60 (250um) | 13.2 | 26.0 | 4.8 | 13.2 | 6.8 | 16.4 | 10.0 | 25.6 | 14.4 | 20.4 |
| 80 (180um) | 10.4 | 36.4 | 4.8 | 18.0 | 10.0 | 26.4 | 8.4 | 34.0 | 40.8 | 61.2 |
| 100 (150um) | 7.6 | 44.0 | 4.8 | 22.8 | 10.4 | 36.8 | 6.4 | 40.4 | 20.0 | 81.2 |
| 200 (75um) | 34.8 | 78.8 | 50.4 | 73.2 | 49.2 | 86.0 | 33.2 | 73.6 | 17.6 | 98.8 |
| 325(45um) | 15.2 | 94.0 | 22.0 | 95.2 | 9.2 | 95.2 | 21.2 | 94.8 | 1.2 | 100 |
| pan (<45um) . | 6.0 | 100 | 4.8 | 100 | 4.8 | 100 | 5.2 | 100 | 0 | 100 |
| Bulk Density g/cc | 0.63 | | 0.54 | | 0.50 | | 0.59 | | 0.48 | |
| Moisture % | 0.50 | | 0.78 | | 0.43 | | 0.46 | | 0.78 | |
| Flow g/sec | 7.6 | | 7.4 | | 7.3 | | 7.1 | | 8.6 | |

**TABLE 1C**

| | EX C1 | | EX C2 | | EX C3 | | EX C4 | |
|---|---|---|---|---|---|---|---|---|
| US Mesh | % R | % CR | % R | % CR | % R | % CR | % R | % CR |
| 20 (850um) | 0 | 0 | 0.4 | 0.4 | 0.8 | 0.8 | 0.4 | 0.4 |
| 40 (425um) | 12.0 | 12.0 | 14.8 | 15.2 | 37.2 | 38.0 | 35.2 | 35.6 |
| 60 (250um) | 18.8 | 30.8 | 18.0 | 33.2 | 20.4 | 58.4 | 24.0 | 59.6 |
| 80 (180um) | 13.2 | 44.0 | 12.4 | 45.6 | 11.6 | 70.0 | 14.8 | 74.4 |
| 100 (150um) | 5.2 | 49.2 | 5.6 | 51.2 | 4.8 | 74.8 | 5.6 | 80.0 |
| 200 (75um) | 19.6 | 68.8 | 18.4 | 69.6 | 13.2 | 88.0 | 14.0 | 94.0 |
| 325 (45um) | 8.8 | 77.6 | 5.6 | 75.2 | 1.2 | 89.2 | 0.4 | 94.4 |
| pan (<45um) | 22.4 | 100 | 24.8 | 100 | 10.8 | 100 | 5.6 | 100 |
| Bulk Density g/cc | 0.71 | | 0.71 | | 0.64 | | 0.64 | |
| Moisture % | 0.59 | | 0.71 | | 0.21 | | 0.31 | |
| Flow g/sec | 6.2 | | 5.2 | | 7.5 * | | 11.3 | |

| | | | | | | | | |
|---|---|---|---|---|---|---|---|---|
| * needed vibration | | | | | | | | |

The tableting performance was evaluated for of the compositions of Examples 1-10 and for Comparison Examples C1-C4. Tablets were made using a tablet press (Manesty Betapress Series 16, Manesty Machines Ltd., U.K.). Tablet hardness was measured using a Schleuniger hardness tester and are reported in kiloponds (kp). Friability and capping were measured using a VanKel friabilator by tumbling 20 tablets for 100 drum rotations. Capping is reported as the percentage of tablets that exhibited cracking and separation. Friability is reported as percentage weight loss, based on weight of particles that did not exhibit capping. Results are set forth in TABLES 2A-2G.

**TABLE 2A**

| | Ex 1 | | | | | Ex 2 | | | | |
|---|---|---|---|---|---|---|---|---|---|---|
| Comp, force (tons) | 0.5 | 1.0 | 1.5 | 2.0 | 2.5 | 0.5 | 1 | 1.5 | 2 | 2.5 |
| Ejection force (lb) | 40 | 50 | 50 | 50 | 50 | 45 | 55 | 60 | 60 | 60 |
| Weight (mg) | 738 | 739 | 740 | 740 | 741 | 740 | 740 | 737 | 740 | 740 |
| Hardness (kp) | 12.3 | 16.0 | 11.8 | 12.8 | 14.9 | 10.3 | 14.5 | 17.0 | 16.5 | 15.9 |
| Thickness (inch) | 0.263 | 0.248 | 0.244 | 0.243 | 0.243 | 0.265 | 0.250 | 0.244 | 0.243 | 0.242 |
| Friability (%) | 0.34 | 0.37 | 0.34 | 0.44 | 0.34 | 0.34 | 0.24 | 0.17 | 0.24 | 0.30 |
| Capping (%) | 0 | 0 | 0 | 0 | 0 | 0 | 0 | 0 | 0 | 0 |

**TABLE 2B**

| | Ex 3 | | | | | Ex 4 | | | | |
|---|---|---|---|---|---|---|---|---|---|---|
| Comp. force (tons) | 0.5 | 1 | 1.5 | 2 | 2.5 | 0.5 | 1.0 | 1.5 | 2.0 | 2.5 |
| Ejection force (lb) | 60 | 70 | 70 | 70 | 65 | 50 | 70 | 75 | 75 | 75 |
| Weight (mg) | 739 | 740 | 740 | 742 | 738 | 741 | 739 | 739 | 740 | 737 |
| Hardness (kp) | 8.7 | 13.7 | 16.3 | 13.1 | 13.2 | 8.3 | 13.4 | 16.4 | 16.8 | 15.5 |
| Thickness (inch) | 0.258 | 0.246 | 0.242 | 0.241 | 0.239 | 0.254 | 0.240 | 0.234 | 0.232 | 0.232 |
| Friability (%) | 0.10 | 0.10 | 0.14 | 0.17 | 0.17 | 0.41 | 0.24 | 0.20 | 0.14 | 0.27 |
| Capping (%) | 0 | 0 | 0 | 0 | 0 | 0 | 0 | 0 | 0 | 0 |

**TABLE 2C**

| | Ex 5 | | | | Ex 6 | | | |
|---|---|---|---|---|---|---|---|---|
| Comp. force (tons) | 1.0 | 1.5 | 2.0 | 2.5 | 1.0 | 1.5 | 2.0 | 2.5 |
| Ejection force (!b) | 80 | 90 | 95 | 95 | 75 | 80 | 85 | 90 |
| Weight (mg) | 741 1 | 740 | 740 | 740 | 740 | 740 | 740 | 740 |
| Hardness (kp) | 12.5 | 15.0 | 18.3 | 17.0 | 11.9 | 14.5 | 18.3 | 16.9 |
| Thickness (inch) | 0.245 | 0.236 | 0.233 | 0.231 | 0.244 | 0.238 | 0.233 | 0.231 |
| Friability (%) | 0.44 | 0.54 | 0.44 | 0.61 | 0.36 | 0.37 | 0.40 | 0.48 |
| Capping (%) | 0 | 0 | 0 | 0 | 0 | 0 | 0 | 0 |

**TABLE 2D**

| | Ex 7 | | | | | Ex 8 | | | |
|---|---|---|---|---|---|---|---|---|---|
| Comp. force (tons) | 0.5 | 1.0 | 1.5 | 2.0 | 2.5 | 0.5 | 1.0 | 1.5 | 2.5 |
| Ejection force (lb) | 40 | 60 | 65 | 65 | 65 | 40 | 55 | 60 | 60 |
| Weight (mg) | 739 | 738 | 737 | 737 | 735 | 738 | 739 | 737 | 736 |
| Hardness (kp) | 7.4 | 13.3 | 15.2 | 14.7 | 13.9 | 7.8 | 13.8 | 16.3 | 14.8 |
| Thickness (inch) | 0.266 | 0.241 | 0.235 | 0.233 | 0.232 | 0.264 | 0.244 | 0.237 | 0.234 |
| Friability (%) | 0.19 | 0.18 | 0.21 | 0.28 | 0.42 | 0.57 | 0.39 | 0.31 | 0.96 |
| Capping (%) | 0 | 0 | 0 | 0 | 0 | 0 | 0 | 0 | 0 |

**TABLE 2E**

| | Ex 9 | | | | | Ex 10 | | | | |
|---|---|---|---|---|---|---|---|---|---|---|
| Comp. force (tons) | 0.5 | 1.0 | 1.5 | 2.0 | 2.5 | 0.5 | 1.0 | 1.5 | 2.0 | 2.5 |
| Ejection force (Ib) | 35 | 55 | 55 | 55 | 55 | 35 | 45 | 45 | 45 | 45 |
| Weight (mg) | 738 | 737 | 735 | 739 | 736 | 737 | 738 | 737 | 738 | 735 |
| Hardness (kp) | 7.8 | 13.7 | 16.9 | 18.3 | 16.1 | 10.6 | 14.2 | 13.4 | 14.0 | 13.1 |
| Thickness (inch) | 0.263 | 0.242 | 0.235 | 0.233 | 0.232 | 0.248 | 0.236 | 0.234 | 0.233 | 0.233 |
| Friability (%) | 0.05 | 0.20 | 0.16 | 0.26 | 0.30 | 0.26 | 0.22 | 0.26 | 0.38 | 0.49 |
| Capping (%) | 0 | 0 | 0 | 0 | 0 | 0 | 0 | 0 | 0 | 0 |

**TABLE 2F**

| | Ex C1 | | | | | Ex C2 | | | | |
|---|---|---|---|---|---|---|---|---|---|---|
| Comp. force (tons) | 0.5 | 1.0 | 1.5 | 2.0 | 2.5 | 0.5 | 1.0 | 1.5 | 2.0 | 2.25 |
| Ejection force (lb) | 50 | 90 | 100 | 100 | 100 | 60 | 90 | 95 | 100 | 80 |
| Weight (mg) | 738 | 741 | 739 | 737 | 739 | 737 | 740 | 738 | 739 | 737 |
| Hardness (kp) | 4.1 | 10.1 | 12.5 | 15.5 | 12.6 | 4.6 | 9.1 | 12.8 | 9.8 | 10.2 |
| Thickness (inch) | 0.268 | 0.246 | 0.247 | 0.234 | 0.232 | 0.264 | 0.247 | 0.246 | 0.246 | 0.242 |
| Friability (%) | 2.35 | 0.40 | 0.27 | 0.37 | 1.00 | 1.76 | 0.41 | 0.24 | 0.47 | |
| Capping (%) | 0 | 0 | 0 | 0 | 0 | 0 | 0 | 0 | 0 | 100 |

**TABLE 2G**

| | Ex C3 | | | | Ex C4 | | | |
|---|---|---|---|---|---|---|---|---|
| Comp. force (tons) | 0.875 | 1.5 | 2.0 | 2.5 | 1.25 | 1.5 | 2.0 | 2.5 |
| Ejection force (lb) | 50 | 60 | 60 | 60 | 60 | 60 | 60 | 55 |
| Weight (mg) | 738 | 738 | 739 | 741 | 740 | 739 | 738 | 741 |
| Hardness (kp) | 11.1 | 15.4 | 16.6 | 13.9 | 12.1 | 13.1 | 11.3 | 11.0 |
| Thickness (inch) | 0.256 | 0.245 | 0.242 | 0.241 | 0.250 | 0.246 | 0.244 | 0.241 |
| Friability (%) | 0.24 | 0.20 | 0.20 | 0.27 | 0.24 | 0.24 | 1.08 | 0.42 |
| Capping (%) | 0 | 0 | 0 | 0 | 0 | 0 | 0 | 35 |

The dissolution properties of tablets made by compressing the composition of Example 1 under a force of 1 ton (hardness of 16.0 kp) and tablets made by compressing the composition of Example 3 under a force of 1.5 tons (hardness of 16.3 kp), as set forth above in TABLE 2A and 2B were measured by the USP dissolution method (US Pharmacopeia) using 900 ml water and paddles rotating at 50 rpm. Dissolved guaifenesin concentration was determined based on UV absorbtion at 274 nm wavelength. Results are set forth in TABLE 3A.

**TABLE 3A**

| | Ex 1 (tableted @ 1.0 ton) | | | | Ex 3 (tableted @ 1.5 tons) | | | |
|---|---|---|---|---|---|---|---|---|
| | tab. wt. (mg.) | 15 min. | 30 min. | 45 min. | tab. wt. (mg.) | 15 min. | 30 min. | 45 min. |
| 1 | 733.9 | 70.8 | 99.6 | 100.0 | 744.6 | 62.5 | 96.0 | 101.1 |
| 2 | 735.9 | 68.1 | 98.7 | 100.2 | 740.8 | 62.7 | 94.2 | 100.7 |
| 3 | 735.2 | 67.4 | 98.7 | 99.8 | 743.7 | 61.4 | 94.6 | 100.7 |
| 4 | 734.7 | 69.2 | 99.8 | 98.0 | 741.7 | 61.4 | 97.5 | 100.7 |
| 5 | 738.6 | 63.2 | 98.2 | 98.4 | 740.3 | 60.7 | 97.3 | 100.7 |
| 6 | 737.4 | 65.6 | 98.4 | 99.3 | 742.5 | 62.0 | 96.6 | 101.1 |
| MIN | 733.9 | 63.2 | 98.2 | 98.0 | 740.3 | 60.7 | 94.2 | 100.7 |
| MAX | 738.6 | 70.8 | 99.8 | 100.2 | 744.6 | 62.7 | 97.5 | 101.1 |
| AVG | 736.0 | 67.4 | 98.9 | 99.3 | 742.3 | 61.8 | 96.0 | 100.8 |

Guaifenesin compositions made according to the method of the present invention provide performance advantages compared to analogous compositions made by other methods. The guaifenesin composition of the present invention provides improved robustness and flexibility with regard to the processing conditions used to produce compressed dosage forms from such compositions, can be processed under relatively low compressive forces to produce compressed dosage forms that exhibit low friability and high hardness and are resistant to capping under relatively high compressive forces. The guaifenesin composition of the present invention also provides improved flow properties and allows more rapid processing of the composition, that is, more rapid flow of the composition allows more rapid transfer of the composition into compression machinery.

## Claims

1. A guaifenesin composition, comprising guaifenesin and a binder and being in the form of particles, wherein by sieve analysis, based on the total weight of the composition, less than about 30 percent by weight of the particles exhibit a particle size of greater than about 425 µm and greater than about 80 percent by weight of the particles exhibit a particle size of greater than about 45 µm.

2. The composition of claim 1, wherein the composition comprises guaifenesin, a binder, a solubilizer, a glidant and a lubricant.

3. The composition of claim 1, wherein the composition comprises guaifenesin, a polyvinylpyrrolidone binder, a maltodextrin, a silica and stearic acid.

4. The composition of claim 1, wherein the composition, based on the total weight of dry ingredients, from 85 to 97.5 percent by weight guaifenesin, from 1.0 to 7 percent by weight of a binder, from 0.2 to 4 percent by weight of a solubilizer or a disintegrant or a solubilizer and a disintegrant, from 0.1 to 2 percent by weight of a glidant, and from 0.1 to 2 percent by weight of a lubricant.

5. The composition of claim 1, wherein the guaifenesin is in the form of particles and wherein by sieve analysis, based on the total weight of the guaifenesin particles, from 10 to 60 percent by weight of the particles exhibit a particle size of from greater than 45 µm to less than 150 µm.

6. The composition of claim 1, wherein by sieve analysis, based on the total weight of the guaifenesin particles, greater than about 10 percent by weight of the guaifenesin particles exhibit a particle size of greater than 75 µm and greater than about 55 percent by weight of the particles exhibit a particle size of greater than 45 µm.

7. The composition of claim 1, wherein less than about 25 percent by weight of the particles exhibit a particle size of greater than about 425 µm greater than about 85 percent by weight of the particles exhibit a particle size of greater than about 45 µm, and from 17 to 55 percent by weight of the particles exhibit a particle size of from greater than 45 µm less than 150 µm.

8. The composition of claim 1, wherein the composition exhibits a flow rate of greater than or equal to 6.5 g per second, as measured using a VanKel flowmeter.

9. A guaifenesin composition, comprising from 85 to 97.5 percent by weight guaifenesin, from 1.0 to 7 percent by weight of a binder, from 0.2 to 4 percent by weight of a solubilizer or a disintegrant or of a solubilizer and a disintegrant, from 0.1 to 2 percent by weight of a glidant, and from 0.1 to 2 percent by weight of a lubricant and being in the form of particles and wherein by sieve analysis, based on the total weight of the composition, less than about 30 percent by weight of the particles exhibit a particle size of greater than about 425 µm, greater than about 80 percent by weight of the particles exhibit a particle size of greater than about 45 µm, and from 10 to 60 percent by weight of the particles exhibit a particle size of from greater than 45 µm to less than 150 µm.

10. A guaifenesin composition, comprising from 85 to 97.5 percent by weight guaifenesin, from 1.0 to 7 percent by weight of a binder, from 0.2 to 4 percent by weight of a solubilizer or a disintegrant or of a solubilizer and a disintegrant, from 0.1 to 2 percent by weight of a glidant, and from 0.1 to 2 percent by weight of a lubricant and being in the form of particles and wherein by sieve analysis, based on the total weight of the composition, less than about 25 percent by weight of the particles exhibit a particle size of greater than about 425 µm, greater than about 85 percent by weight of the particles exhibit a particle size of greater than about 45 µm, and from 17 to 55 percent by weight of the particles exhibit a particle size of from greater than 45 micrometers to less than 150 µm.

11. A method for making a compressible guaifenesin composition according to claim 1, comprising:
mixing a mixture comprising guaifenesin, a binder and water to form agglomerates;
drying the agglomerates to form dried particles;
classifying the dried particles into first particles having particle sizes less than or equal to a selected classification limit and second particles having particle sizes greater than the classification limit;
milling the second dried particles to reduce their size to less than the classification limit; and
combining the milled second particles with the first particles to form the guaifenesin composition.

12. The method of claim 11, wherein the mixing under high shear conditions is conducted in a vertical high shear mixer.

13. The method of claim 11, wherein, prior to drying, the agglomerates are wet milled under conditions effective to reduce the size of the agglomerates.

14. The method of claim 11, wherein the drying is conducted at less than 65°C.

15. The method of claim 11, wherein the drying is conducted in a fluidized bed at less than 65°C and a fluidization gas flow rate of from 36,8 m³ (1300 cubic feet) to 142 m³ (5000 cubic feet) per minute.

16. The method of claim 11, wherein the classification limit is greater than or equal to 850 µm.

17. The method of claim 11, wherein the classification limit is greater than or equal to 1.18 millimeters.

18. The method of claim 11, wherein the second dried particles are dry milled in a conical mill.

19. The method of claim 11, wherein by sieve analysis, based on the total weight of the guaifenesin particles, from 10 to 60 percent by weight of the particles exhibit a particle size of from greater than 45 µm to less than 150 µm.

20. A method for making a compressible guaifenesin composition, comprising:
mixing a mixture comprising guaifenesin, a binder, a solubilizer or a disintegrant and water to form agglomerates,
wet milling the agglomerates,
drying the wet milled agglomerates to form dried particles,
classifying the dried particles into first particles having a particle size of less or equal to a selected classification limit of greater than 850 µm and second particles having a particle size greater than the classification limit,
dry milling the second dried particles to reduce their size to less than the classification limit, and
combining the milled second particles with the first particles to form the guaifenesin composition and wherein by sieve analysis, based on the total weight of the composition, less than about 30 percent by weight of the particles of the composition exhibit a particle size of greater than about 425 µm greater than about 80 percent by weight of the particles of the composition exhibit a particle size of greater than about 45 µm, and from 10 to 60 percent by weight of the particles of the composition exhibit a particle size of from greater than 45 µm to less than 150 µm.

21. A method for making a guaifenesin dosage form, comprising compressing a guaifenesin composition, said composition comprising guaifenesin and a binder and being in the form of particles and wherein by sieve analysis, based on the total weight of the composition, less than about 30 percent by weight of the particles exhibit a particle size of greater than about 425 µm and greater than about 80 percent by weight of the particles exhibit a particle size of greater than about 45 µm.

22. The method of claim 21, wherein by sieve analysis, based on the total weight of the composition, and from 10 to 60 percent by weight of the particles exhibit a particle size of from greater than 45 micrometers to less than 150 µm.

23. The method of claim 21, wherein the guaifenesin composition exhibits a flow rate of greater than or equal to 6.5 grams per second, as measured without vibration using a VanKel flowmeter.

24. The method of claim 21, wherein guaifenesin dosage forms made by compressing the guaifenesin composition at a compressive force of less than or equal to 1.5 tons exhibit a hardness of greater than 15.0 kiloponds.

25. The method of claim 21, wherein guaifenesin dosage forms made by compressing the guaifenesin composition at a compressive force of less than or equal to 2.5 tons exhibit substantially no capping.

26. The method of claim 21, wherein guaifenesin dosage forms made by compressing the guaifenesin composition at a compressive force of from about 0.5 ton to 2.5 tons exhibit less than 1.0% friability.

27. The method of claim 21, wherein prior to the step of compressing, the guaifenesin composition is blended with one or more co-active ingredient selected from pseudoephedrine hydrochloride, dextromethorphan hydrobromide, chlorpheniramine maleate and acetaminophen.

28. A method for making a guaifenesin dosage form, comprising compressing a guaifenesin composition, said composition comprising guaifenesin, a binder, a solubilizer, a glidant and a lubricant and being in the form of particles wherein by sieve analysis, based on the total weight of the composition, less than about 30 percent by weight of the particles exhibit a particle size of greater than about 425 µm and greater than about 80 percent by weight of the particles exhibit a particle size of greater than about 45 µm, and from 10 to 60 percent by weight of the particles exhibit a particle size of from greater than 45 µm to less than 150 µm, wherein the composition exhibits a flow rate of greater than or equal to 6.5 grams per second, as measured without vibration using a VanKel flowmeter, wherein dosage forms made by compressing the composition at a compressive force of less than or equal to 1.5 tons exhibit a hardness of greater than 15.0 kiloponds, wherein dosage forms made by compressing the composition at a compressive force of less than or equal to 2.5 tons exhibit substantially no capping and wherein dosage forms made by compressing the composition at a compressive force of from 0.5 ton to 2.5 tons exhibit less than 1.0% friability.

## Patentansprüche

1. Guaifenesin-Zusammensetzung, die Guaifenesin und ein Bindemittel umfasst und in Form von Teilchen vorliegt, wobei gemäß Siebanalyse, bezogen auf das Gesamtgewicht der Zusammensetzung weniger als etwa 30 Gew.-% der Teilchen eine Teilchengröße von größer als etwa 425 µm aufweisen und mehr als etwa 80 Gew.-% der Teilchen eine Teilchengröße von größer als etwa 45 µm aufweisen.

2. Zusammensetzung nach Anspruch 1, wobei die Zusammensetzung Guaifenesin, ein Bindemittel, einen Lösungsvermittler, ein Gleitmittel und ein Schmiermittel umfasst.

3. Zusammensetzung nach Anspruch 1, wobei die Zusammensetzung Guaifenesin, ein Polyvinylpyrrolidon-Bindemittel, ein Maltodextrin, ein Siliciumdioxid und Stearinsäure umfasst.

4. Zusammensetzung nach Anspruch 1, wobei die Zusammensetzung, bezogen auf das Gesamtgewicht der trockenen Bestandteile 85 bis 97,5 Gew.-% Guaifenesin, 1,0 bis 7 Gew.-% eines Bindemittels, 0,2 bis 4 Gew.-% eines Lösungsvermittlers oder eines Sprengmittels oder eines Lösungsvermittlers und eines Sprengmittels, 0,1 bis 2 Gew.-% eines Gleitmittels und 0,1 bis 2 Gew.-% eines Schmiermittels umfasst.

5. Zusammensetzung nach Anspruch 1, wobei das Guaifenesin in der Form von Teilchen vorliegt und wobei gemäß Siebanalyse, bezogen auf das Gesamtgewicht der Guaifenesinteilchen, 10 bis 60 Gew.-% der Teilchen eine Teilchengröße von größer als 45 µm bis weniger als 150 µm aufweisen.

6. Zusammensetzung nach Anspruch 1, wobei gemäß Siebanalyse, bezogen auf das Gesamtgewicht der Guaifenesinteilchen, mehr als etwa 10 Gew.-% der Guaifenesinteilchen eine Teilchengröße von größer als 75 µm aufweisen und mehr als etwa 55 Gew.-% der Teilchen eine Teilchengröße von größer als 45 µm aufweisen.

7. Zusammensetzung nach Anspruch 1, wobei weniger als etwa 25 Gew.-% der Teilchen eine Teilchengröße von größer als etwa 425 µm aufweisen, mehr als etwa 85 Gew.-% der Teilchen eine Teilchengröße von größer als etwa 45 µm aufweisen und 17 bis 55 Gew.-% der Teilchen eine Teilchengröße von größer als 45 µm bis weniger als 150 µm aufweisen.

8. Zusammensetzung nach Anspruch 1, wobei die Zusammensetzung eine Fließgeschwindigkeit von größer als oder gleich 6,5 g pro Sekunde, gemessen unter Verwendung eines VanKel-Fließmessers, aufweist.

9. Guaifenesin-Zusammensetzung, die 85 bis 97,5 Gew.-% Guaifenesin, 1,0 bis 7 Gew.-% eines Bindemittels, 0,2 bis 4 Gew.-% eines Lösungsvermittlers oder eines Sprengmittels oder eines Lösungsvermittlers und eines Sprengmittels, 0,1 bis 2 Gew.-% eines Gleitmittels und 0,1 bis 2 Gew.-% eines Schmiermittels, umfasst und in der Form von Teilchen vorliegt und wobei gemäß Siebanalyse, bezogen auf das Gesamtgewicht der Zusammensetzung, weniger als etwa 30 Gew.-% der Teilchen eine Teilchengröße von größer als etwa 425 µm aufweisen, mehr als etwa 80 Gew.-% der Teilchen eine Teilchengröße von größer als etwa 45 µm aufweisen und 10 bis 60 Gew.-% der Teilchen eine Teilchengröße von größer als 45 µm bis weniger als 150 µm aufweisen.

10. Guaifenesin-Zusammensetzung, die 85 bis 97,5 Gew.-% Guaifenesin, 1,0 bis 7 Gew.-% eines Bindemittels, 0,2 bis 4 Gew.-% eines Lösungsvermittlers oder eines Sprengmittels oder eines Lösungsvermittlers und eines Sprengmittels, 0,1 bis 2 Gew.-% eines Gleitmittels und 0,1 bis 2 Gew.-% eines Schmiermittels umfasst, und in der Form von Teilchen vorliegt und wobei gemäß Siebanalyse, bezogen auf das Gesamtgewicht der Zusammensetzung, weniger als etwa 25 Gew.-% der Teilchen eine Teilchengröße von größer als etwa 425 µm aufweisen, mehr als etwa 85 Gew.-% der Teilchen eine Teilchengröße von größer als etwa 45 µm aufweisen und 17 bis 55 Gew.-% der Teilchen eine Teilchengröße von größer als 45 Mikrometer bis weniger als 150 µm aufweisen.

11. Verfahren zur Herstellung einer kompressiblen Guaifenesin-Zusammensetzung nach Anspruch 1, umfassend:
das Mischen eines Gemisches, umfassend Guaifenesin, ein Bindemittel und Wasser, um Agglomerate zu bilden;
das Trocknen der Agglomerate, um getrocknete Teilchen zu bilden;
das Klassieren der getrockneten Teilchen in erste Teilchen mit Teilchengrößen, die kleiner als oder gleich ein ausgewählter Klassierungsgrenzwert sind und in zweite Teilchen mit Teilchengrößen, die größer als der Klassierungsgrenzwert sind;
das Vermahlen der zweiten getrockneten Teilchen, um deren Größe auf einen kleineren Wert als den Klassierungsgrenzwert zu verringern; und
das Vereinigen der vermahlenen zweiten Teilchen mit den ersten Teilchen, um die Guaifenesin-Zusammensetzung zu bilden.

12. Verfahren nach Anspruch 11, wobei das Vermischen unter Bedingungen hoher Scherkräfte in einem vertikalen Mischer mit hoher Scherkraft durchgeführt wird.

13. Verfahren nach Anspruch 11, wobei die Agglomerate vor dem Trocknen nass vermahlen werden, und zwar unter Bedingungen, die eine Verringerung der Größe der Agglomerate bewirken.

14. Verfahren nach Anspruch 11, wobei das Trocknen bei weniger als 65°C durchgeführt wird.

15. Verfahren nach Anspruch 11, wobei das Trocknen in einem Fließbett bei weniger als 65°C und einer Strömungsgeschwindigkeit des fluidisierenden Gases von 36,8 m³ (1300 Kubikfuß) bis 142 m³ (5000 Kubikfuß) pro Minute durchgeführt wird.

16. Verfahren nach Anspruch 11, wobei der Klassierungsgrenzwert größer als oder gleich 850 µm ist.

17. Verfahren nach Anspruch 11, wobei der Klassierungsgrenzwert größer als oder gleich 1,18 mm ist.

18. Verfahren nach Anspruch 11, wobei die zweiten getrockneten Teilchen in einer Konusmühle trocken vermahlen werden.

19. Verfahren nach Anspruch 11, wobei gemäß Siebanalyse, bezogen auf das Gesamtgewicht der Guaifenesinteilchen, 10 bis 60 Gew.-% der Teilchen eine Teilchengröße von größer als 45 µm bis weniger als 150 µm aufweisen.

20. Verfahren zur Herstellung einer kompressiblen Guaifenesin-Zusammensetzung, umfassend:
das Vermischen eines Gemisches, umfassend Guaifenesin, ein Bindemittel, einen Lösungsvermittler oder ein Sprengmittel und Wasser, um Agglomerate zu bilden;
das Nassvermahlen der Agglomerate,
das Trocknen der nass vermahlenen Agglomerate, um getrocknete Teilchen zu bilden;
das Klassieren der getrockneten Teilchen in erste Teilchen mit einer Teilchengröße von weniger als oder gleich einem ausgewählten Klassierungsgrenzwert von größer als 850 µm und in zweite Teilchen mit einer Teilchengröße, die größer als der Klassierungsgrenzwert ist;
das trockene Vermahlen der zweiten Teilchen, um deren Größe auf einen Wert, der kleiner als der Klassierungsgrenzwert ist, zu verringern; und
das Vereinigen der vermahlenen zweiten Teilchen mit den ersten Teilchen, um die Guaifenesin-Zusammensetzung zu bilden, und wobei gemäß Siebanalyse, bezogen auf das Gesamtgewicht der Zusammensetzung, weniger als etwa 30 Gew.-% der Teilchen der Zusammensetzung eine Teilchengröße von größer als etwa 425 µm aufweisen, mehr als etwa 80 Gew.-% der Teilchen der Zusammensetzung eine Teilchengröße von größer als etwa 45 µm aufweisen und 10 bis 60 Gew.-% der Teilchen der Zusammensetzung eine Teilchengröße von größer als 45 µm bis weniger als 150 µm aufweisen.

21. Verfahren zur Herstellung einer Guaifenesin-Dosierungsform, umfassend das Komprimieren einer Guaifenesin-Zusammensetzung, wobei die Zusammensetzung Guaifenesin und ein Bindemittel umfasst, und in der Form von Teilchen vorliegt, und wobei gemäß Siebanalyse, bezogen auf das Gesamtgewicht der Zusammensetzung, weniger als etwa 30 Gew.-% der Teilchen eine Teilchengröße von größer als etwa 425 µm aufweisen und mehr als etwa 80 Gew.-% der Teilchen eine Teilchengröße von größer als etwa 45 µm aufweisen.

22. Verfahren nach Anspruch 21, wobei gemäß Siebanalyse, bezogen auf das Gesamtgewicht der Zusammensetzung, 10 bis 60 Gew.-% der Teilchen eine Teilchengröße von größer als 45 Mikrometer bis weniger als 150 µm aufweisen.

23. Verfahren nach Anspruch 21, wobei die Guaifenesin-Zusammensetzung eine Fließgeschwindigkeit von größer als oder gleich 6,5 g pro Sekunde, gemessen ohne Vibration unter Verwendung eines VanKel-Fließmessers, aufweist.

24. Verfahren nach Anspruch 21, wobei die Guaifenesin-Dosierungsformen, die durch Komprimieren der Guaifenesin-Zusammensetzung bei einer Kompressionskraft von weniger als oder gleich 1,5 t hergestellt worden sind, eine Härte von größer als 15,0 Kilopond aufweisen.

25. Verfahren nach Anspruch 21, wobei die Guaifenesin-Dosierungsformen, die durch Komprimieren der Guaifenesin-Zusammensetzung bei einer Kompressionskraft von weniger als oder gleich 2,5 t hergestellt worden sind, im Wesentlichen keine Kappung aufweisen.

26. Verfahren nach Anspruch 21, wobei die Guaifenesin-Dosierungsformen, die durch Komprimieren der Guaifenesin-Zusammensetzung bei einer Kompressionskraft von etwa 0,5 t bis etwa 2,5 t hergestellt worden sind, eine Brüchigkeit von weniger als 1,0 % aufweisen.

27. Verfahren nach Anspruch 21, wobei die Guaifenesin-Zusammensetzung vor der Stufe des Komprimierens mit einem oder mehreren co-aktiven Bestandteilen ausgewählt aus Pseudoeffedrinhydrochlorid, Dextrometorphanhydrobromid, Chlorpheniraminmaleat und Acetaminophen, vermischt wird.

28. Verfahren zur Herstellung einer Guaifenesin-Dosierungsform, umfassend das Komprimieren einer Guaifenesin-Zusammensetzung, wobei die Zusammensetzung Guaifenesin, ein Bindemittel, einen Lösungsvermittler, ein Gleitmittel und ein Schmiermittel umfasst, und in der Form von Teilchen vorliegt, wobei gemäß Siebanalyse, bezogen auf das Gesamtgewicht der Zusammensetzung, weniger als etwa 30 Gew.-% der Teilchen eine Teilchengröße von größer als etwa 425 µm aufweisen und mehr als etwa 80 Gew.-% der Teilchen eine Teilchengröße von größer als 45 µm aufweisen und 10 bis 60 Gew.-% der Teilchen eine Teilchengröße von größer als 45 µm bis weniger als 150 µm aufweisen, wobei die Zusammensetzung eine Fließgeschwindigkeit von größer als oder gleich 6,5 g pro Sekunde, gemessen ohne Vibration unter Verwendung eines VanKel-Fließmessers, aufweist, wobei die Dosierungsformen die durch Komprimieren der Zusammensetzung bei einer Kompressionskraft von weniger als oder gleich 1,5 t hergestellt worden sind, eine Härte von größer als 15,0 Kilopond aufweisen, wobei die Dosierungsformen, die durch Komprimieren der Zusammensetzung bei einer Kompressionskraft von weniger als oder gleich 2,5 t hergestellt worden sind, im Wesentlichen keine Kappung aufweisen, und wobei die Dosierungsformen, die durch Komprimieren der Zusammensetzung bei einer Kompressionskraft von 0,5 t bis 2,5 t hergestellt worden sind, weniger als 1,0 % Brüchigkeit aufweisen.

## Revendications

1. Composition de guaïfénésine, comprenant de la guaïfénésine et un liant et étant sous la forme de particules, dans laquelle, par analyse granulométrique, sur la base du poids total de la composition, moins d'environ 30 pour cent en poids des particules présentent une taille supérieure à environ 425 µm et plus d'environ 80 pour cent en poids des particules présentent une taille supérieure à environ 45 µm.

2. Composition selon la revendication 1, dans laquelle la composition comprend de la guaïfénésine, un liant, un solubilisant, un agent de glissement et un lubrifiant.

3. Composition selon la revendication 1, dans laquelle la composition comprend de la guaïfénésine, un liant polyvinylpyrrolidone, une maltodextrine, une silice et de l'acide stéarique.

4. Composition selon la revendication 1, dans laquelle la composition, sur la base du poids total des ingrédients secs, comprend de 85 à 97,5 pour cent en poids de guaïfénésine, de 1,0 à 7 pour cent en poids d'un liant, de 0,2 à 4 pour cent en poids d'un solubilisant ou d'un désintégrant ou d'un solubilisant et d'un désintégrant, de 0,1 à 2 pour cent en poids d'un agent de glissement, et de 0,1 à 2 pour cent en poids d'un lubrifiant.

5. Composition selon la revendication 1, dans laquelle la guaïfénésine est sous la forme de particules et dans laquelle, par analyse granulométrique, sur la base du poids total des particules de guaïfénésine, de 10 à 60 pour cent en poids des particules présentent une taille supérieure à 45 µm et inférieure à 150 µm.

6. Composition selon la revendication 1, dans laquelle, par analyse granulométrique, sur la base du poids total des particules de guaïfénésine, plus d'environ 10 pour cent en poids des particules de guaïfénésine présentent une granulométrie supérieure à 75 µm et plus d'environ 55 pour cent en poids des particules présentent une taille supérieure à 45 µm.

7. Composition selon la revendication 1, dans laquelle moins d'environ 25 pour cent en poids des particules présentent une taille supérieure à environ 425 µm, plus d'environ 85 pour cent en poids des particules présentent une taille supérieure à environ 45 µm, et 17 à 55 pour cent en poids des particules présentent une taille supérieure à 45 µm et inférieure à 150 µm.

8. Composition selon la revendication 1, dans laquelle la composition présente un débit d'écoulement supérieur ou égal à 6,5 g par seconde, tel que mesuré au moyen d'un débitmètre VanKel.

9. Composition de guaïfénésine comprenant de 85 à 97,5 pour cent en poids de guaïfénésine, de 1,0 à 7 pour cent en poids d'un liant, de 0,2 à 4 pour cent en poids d'un solubilisant ou d'un désintégrant ou d'un solubilisant et d'un désintégrant, de 0,1 à 2 pour cent en poids d'un agent de glissement, et de 0,1 à 2 pour cent en poids d'un lubrifiant, et étant sous la forme de particules, et dans laquelle, par analyse granulométrique, sur la base du poids total de la composition, moins d'environ 30 pour cent en poids des particules présentent une taille supérieure à environ 425 µm, plus d'environ 80 pour cent en poids des particules présentent une taille supérieure à environ 45 µm, et 10 à 60 pour cent en poids des particules présentent une taille supérieure à 45 µm et inférieure à 150 µm.

10. Composition de guaïfénésine comprenant de 85 à 97,5 pour cent en poids de guaïfénésine, de 1,0 à 7 pour cent en poids d'un liant, de 0,2 à 4 pour cent en poids d'un solubilisant ou d'un désintégrant ou d'un solubilisant et d'un désintégrant, de 0,1 à 2 pour cent en poids d'un agent de glissement, et de 0,1 à 2 pour cent en poids d'un lubrifiant, et étant sous la forme de particules, et dans laquelle, par analyse granulométrique, sur la base du poids total de la composition, moins d'environ 25 pour cent en poids des particules présentent une taille supérieure à environ 425 µm, plus d'environ 85 pour cent en poids des particules présentent une taille supérieure à environ 45 µm, et 17 à 55 pour cent en poids des particules présentent une taille supérieure à 45 µm et inférieure à 150 µm.

11. Procédé pour préparer une composition de guaïfénésine compressible selon la revendication 1, comprenant :
le mélange d'un mélange comprenant de la guaïfénésine, un liant et de l'eau pour former des agglomérats ;
le séchage des agglomérats pour former des particules séchées ;
le tri des particules séchées en premières particules ayant des tailles inférieures ou égales à une limite de tri sélectionnée et en deuxièmes particules ayant des tailles supérieures à la limite de tri ;
le broyage des deuxièmes particules séchées pour réduire leur taille à une valeur inférieure à la limite de classification ; et
la combinaison des deuxièmes particules broyées avec les premières particules pour former la composition de guaïfénésine.

12. Procédé selon la revendication 11, dans lequel le mélange dans des conditions de fort cisaillement est effectué dans un mélangeur vertical à fort cisaillement.

13. Procédé selon la revendication 11, dans lequel, avant le séchage, les agglomérats sont broyés à l'état humide dans des conditions efficaces pour réduire la taille des agglomérats.

14. Procédé selon la revendication 11, dans lequel le séchage est effectué à moins de 65°C.

15. Procédé selon la revendication 11, dans lequel le séchage est effectué dans un lit fluidisé à moins de 65°C et à un débit d'écoulement du gaz de fluidisation de 36,8 m³ (1300 pieds cube) à 142 m³ (5000 pieds cube) par minute.

16. Procédé selon la revendication 11, dans lequel la limite de tri est supérieure ou égale à 850 µm.

17. Procédé selon la revendication 11, dans lequel la limite de tri est supérieure ou égale à 1,18 millimètres.

18. Procédé selon la revendication 11, dans lequel les deuxièmes particules séchées sont broyées à l'état sec dans un broyeur conique.

19. Procédé selon la revendication 11, dans lequel, par analyse granulométrique, sur la base du poids total des particules de guaïfénésine, 10 à 60 pour cent en poids des particules présentent une taille supérieure à 45 µm et inférieure à 150 µm.

20. Procédé pour préparer une composition de guaïfénésine compressible, comprenant :
le mélange d'un mélange comprenant de la guaïfénésine, un liant, un solubilisant ou un désintégrant et de l'eau pour former des agglomérats,
le broyage à l'état humide des agglomérats,
le séchage des agglomérats broyés humides pour former des particules séchées,
le tri des particules séchées en premières particules ayant une taille inférieure ou égale à une limite de tri sélectionnée supérieure à 850 µm et en deuxièmes particules ayant une taille supérieure à la limite de tri,
le broyage à l'état sec des deuxièmes particules séchées pour réduire leur taille à une valeur inférieure à la limite de tri, et
la combinaison des deuxièmes particules broyées avec les premières particules pour former la composition de guaïfénésine,
et dans lequel, par analyse granulométrique, sur la base du poids total de la composition, moins d'environ 30 pour cent en poids des particules de la composition présentent une taille supérieure à environ 425 µm, plus d'environ 80 pour cent en poids des particules de la composition présentent une taille supérieure à environ 45 µm, et 10 à 60 pour cent en poids des particules de la composition présentent une granulométrie supérieure à 45 µm et inférieure à 150 µm.

21. Procédé pour préparer une forme posologique de guaïfénésine, comprenant la compression d'une composition de guaïfénésine, ladite composition comprenant de la guaïfénésine et un liant et étant sous la forme de particules, et dans lequel, par analyse granulométrique, sur la base du poids total de la composition, moins d'environ 30 pour cent en poids des particules présentent une taille supérieure à environ 425 µm et plus d'environ 80 pour cent en poids des particules présentent une taille supérieure à environ 45 µm.

22. Procédé selon la revendication 21, dans lequel, par analyse granulométrique, sur la base du poids total de la composition, 10 à 60 pour cent en poids des particules présentent une taille supérieure à 45 micromètres et inférieure à 150 µm.

23. Procédé selon la revendication 21, dans lequel la composition de guaïfénésine présente un débit d'écoulement, mesuré sans vibration au moyen d'un débitmètre VanKel, supérieur ou égal à 6,5 g par seconde,

24. Procédé selon la revendication 21, dans lequel les formes posologiques de guaïfénésine préparées par compression de la composition de guaïfénésine sous une force compressive inférieure ou égale à 1,5 tonnes présentent une dureté supérieure à 15,0 kilogrammes-poids.

25. Procédé selon la revendication 21, dans lequel les formes posologiques de guaïfénésine préparées par compression de la composition de guaïfénésine sous une force compressive inférieure ou égale à 2,5 tonnes ne présentent pratiquement pas de décalottage.

26. Procédé selon la revendication 21, dans lequel les formes posologiques de guaïfénésine préparées par compression de la composition de guaïfénésine sous une force compressive d'environ 0,5 tonne à 2,5 tonnes présentent une friabilité inférieure à 1,0 pour cent.

27. Procédé selon la revendication 21, dans lequel, avant l'étape de compression, la composition de guaïfénésine est mélangée avec un ou plusieurs ingrédients co-actifs choisis parmi le chlorhydrate de pseudo-éphédrine, le bromhydrate de dextrométhorphan, le maléate de chlorphéniramine et l'acétaminophen.

28. Procédé pour préparer une forme posologique de guaïfénésine, comprenant la compression d'une composition de guaïfénésine, ladite composition comprenant de la guaïfénésine, un liant, un solubilisant, un agent de glissement et un lubrifiant et étant sous la forme de particules, dans lequel, par analyse granulométrique, sur la base du poids total de la composition, moins d'environ 30 pour cent en poids des particules présentent une taille supérieure à environ 425 µm et plus d'environ 80 pour cent en poids des particules présentent une taille supérieure à environ 45 µm, et 10 à 60 pour cent en poids des particules présentent une taille supérieure à 45 µm et inférieure à 150 µm, dans lequel la composition présente un débit d'écoulement supérieur ou égal à 6,5 g par seconde, tel que mesuré sans vibration au moyen d'un débitmètre VanKel, dans lequel les formes posologiques préparées par compression de la composition sous une force compressive inférieure ou égale à 1,5 tonnes présentent une dureté supérieure à 15,0 kilogrammes-poids, dans lequel les formes posologiques préparées par compression de la composition sous une force compressive inférieure ou égale à 2,5 tonnes ne présentent pratiquement pas de décalottage, et dans lequel les formes posologiques préparées par compression de la composition sous une force compressive d'environ 0,5 tonne à 2,5 tonnes présentent une friabilité inférieure à 1,0 %.
